(19) **Europäisches Patentamt**
**European Patent Office**
**Office européen des brevets**

(11) **EP 3 253 342 B1**

(12) **EUROPEAN PATENT SPECIFICATION**

(45) Date of publication and mention
of the grant of the patent:
**28.11.2018 Bulletin 2018/48**

(21) Application number: **16703054.3**

(22) Date of filing: **02.02.2016**

(51) Int Cl.:
*A61F 5/443* *(2006.01)*  *A61F 5/448* *(2006.01)*
*A61F 5/449* *(2006.01)*  *A61L 15/00* *(2006.01)*
*A61L 28/00* *(2006.01)*  *A61L 24/00* *(2006.01)*
*A61L 24/04* *(2006.01)*  *A61L 24/06* *(2006.01)*

(86) International application number:
**PCT/DK2016/050028**

(87) International publication number:
**WO 2016/124203 (11.08.2016 Gazette 2016/32)**

(54) **OSTOMY DEVICE**

OSTOMIEVORRICHTUNG

DISPOSITIF D'OSTOMIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.02.2015 DK 201570056
24.11.2015 DK 201570752**

(43) Date of publication of application:
**13.12.2017 Bulletin 2017/50**

(73) Proprietor: **Coloplast A/S
3050 Humblebæk (DK)**

(72) Inventor: **STROEBECH, Esben
2970 Hoersholm (DK)**

(56) References cited:
**EP-A1- 2 012 718    EP-A1- 2 371 920
EP-A1- 2 654 633    WO-A1-2009/006902**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

Disclosed is an ostomy device with an adhesive wafer for attachment to a skin surface of a user and a collecting bag connected to the adhesive wafer. The adhesive wafer typically includes a backing layer, a switchable adhesive composition, an absorbent adhesive composition, and a release liner. WO 2009/006902 A1 is regarded as the closest prior art.

**Background**

In connection with surgery for a number of diseases in the gastro-intestinal tract, one of the consequences in many cases is that the patient is left with an abdominal stoma, such as a colostomy, an ileostomy or a urostomy, in the abdominal wall for the discharge of visceral contents. The discharge of visceral contents cannot be regulated at will. For that purpose, the user will have to rely on an appliance to collect the material emerging from such opening in a bag, which is later emptied and/or discarded at a suitable time. Ostomy appliances are typically attached to the skin of the ostomy user by means of an adhesive wafer on the ostomy appliance.

**Brief Description of the Drawings**

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Figure 1 is a schematic cross-section view of one half of the adhesives of an adhesive wafer not belonging to the invention.

Figure 2 is a schematic cross-section view of one half of the adhesives of an adhesive wafer belonging to the invention.

Figure 3 is a schematic cross-section view of an ostomy device, including an adhesive wafer and a collecting bag.

**Detailed Description of the Invention**

Embodiments provide an ostomy device including an adhesive wafer for attachment to a skin surface of a user, and a collecting bag connected to the adhesive wafer. The collecting bag is suitable for collecting output from the stoma of the user. The adhesive wafer has a through-going hole for accommodating the stoma of the user. In this way, the output from the stoma ends up in the collecting bag. The adhesive wafer includes a backing layer, a first switchable adhesive composition, a second absorbent adhesive composition, and a release liner.

In embodiments, the adhesive wafer will have a proximal ("skin-facing") surface, which faces the skin of the user during use, and a distal ("non-skin-facing") surface, which faces away from the user's skin during use. Before use, the proximal surface of the adhesive wafer can be covered by a release liner, which is releasably attached to the adhesive. The release liner can be removed by the user immediately prior to application of the adhesive wafer to the skin. Both before and during use, the distal surface of the adhesive wafer can be made up of a backing layer, which can be used to attach the collecting bag to the adhesive wafer, for instance by welding. As such, the adhesive wafer may comprise a distal backing layer and a proximal release liner, with the first switchable adhesive composition and the second absorbent adhesive composition located between the backing layer and the release liner.

An ostomy device can be a one-piece appliance comprising a) a base plate (also referred to as a body-side member or face plate) attachable around the stomal opening; and comprising b) a collecting bag attached to the base plate.

On ostomy device can be a two-piece appliance comprising a) a base plate (also referred to as a body-side member) attachable around the stomal opening; and comprising b) a separate collecting bag attachable to the base plate. In this two-piece configuration, the collecting bag can be replaced without replacing the base-plate attached to the skin around the stomal opening. The separate collecting bag may be attached to the body side member in any convenient manner known per se, e.g., via a mechanical coupling, such as a coupling ring, or by an adhesive flange.

The adhesive wafer includes a switchable adhesive composition. Switchable means that the adhesive can be switched between at least two different states with different properties. Switchable adhesives compositions are, for example, known from EP 2 372 920 A1.

The switch is the transition from one state to another state of a switchable composition. The duration of the switch will vary depending on, e.g., the nature of the switch initiator and the method of activation of the switch initiator. Generally, the switch will be a gradual process with a gradual change of physical properties of the material from one state to another state. In some instances, the switch will be very fast and the physical properties will change very quickly, e.g. within seconds, to those of the second state. In other instances, the switch will be slower and the change in properties will happen gradually over a period of, e.g., several minutes or even hours.

In embodiments, the switchable adhesive composition can be switched from a tacky state to a non-tacky or low-tack state in which the switched adhesive has a reduced peel strength relative to the peel strength of the adhesive before switching. In embodiments, the switchable adhesive composition is switchable from a high-tack state with a first peel force to a lower-tack state with a second peel force. In embodiments, the first peel force is higher than the second peel force. The peel force can be measured, e.g., by the 90-degree peel test described herein.

Since the switchable adhesive composition can be switched to a non-tacky or low-tack state, it can initially be provided in a high-tack state with a tack that would otherwise not be suitable for use on skin because it would be too difficult or too painful to remove. In other words, the pre-switch tack can be very high because it is not required that the adhesive can be removed again from the skin in the pre-switch state. In this manner, the switchable adhesive composition can be made to have a pre-switch application state, in which the properties are suitable for application to the skin of the user, and a post-switch removal state, in which the properties are suitable for removing the adhesive from the skin.

Recognizing that the expression "low-tack" is a relative term, it will be defined here as meaning the condition of minimum tackiness which the adhesive reaches after switching from its tacky state. The reduction in peel force may be as great as 100% or as little as 30%. In embodiments, the reduction in peel force is at least 50%. In embodiments, the reduction in peel force is 30-40%, 30-50%, 30-60%, 30-70%, 30-80%, 30-90%, 30-100%, 40-50%, 40-60%, 40-70%, 40-80%, 40-90%, 40-100%, 50-60%, 50-70%, 50-80%, 50-90%, 50-100%, 60-70%, 60-80%, 60-90%, 60-100%, 70-80%, 70-90%, 70-100%, 80-90%, 80-100%, or 90-100%.

The adhesive wafer includes an absorbent adhesive composition. The absorbent adhesive composition is capable of absorbing moisture. The purpose of having an absorbent adhesive composition as a part of an ostomy device is to allow the absorbent adhesive composition to absorb moisture produced by the skin and thereby prevent accumulation of moisture at the skin surface, underneath the ostomy device. Accumulation of moisture on the skin surface can lead to damage of the skin, such as maceration.

By providing an adhesive wafer having both a switchable adhesive composition and an absorbent adhesive composition, the present inventors have been able to construct an ostomy device, which can adhere quickly and strongly to the skin of the user and at the same time properly absorb moisture to prevent damage to the skin underneath the adhesive.

The fast and strong adhesion to the skin effected by the switchable adhesive composition further leads to prevention of leakage of output from the ostomy.

Leakage is when ostomy output makes its way to the skin and clothes outside the ostomy device. This can of course also be damaging to the skin and the adhesive and is, obviously, also problematic to the user in terms of discomfort and smell. Leakage typically results from ostomy output having first leaked into the adhesive and then through the adhesive to the outside, or into the space between the skin and the adhesive and then to the outside. As such, preventing leakage between the adhesive and the skin as well as into the adhesive will also prevent most types of leakage outside of the ostomy device. A special kind of leakage is when the ostomy bag partly or entirely detaches from the skin of the user during use, thus causing the output in the collecting bag to directly spill out. This type of leakage is best prevented by ensuring a strong a durable adhesive bond between the skin of the user and the ostomy device. Also, detachment will typically be a result of the adhesive having been weakened during the course of use, for instance by being affected by the output. As such, ensuring a strong and enduring adhesive bond and preventing leakage will also minimize the risk of the adhesive detaching from the skin and the bag falling off.

By using a switchable adhesive composition with a pre-switch high-tack state, a quick initial adherence between the adhesive and the skin of the user can take place. Such a quick and strong adhesion will, already from application of the adhesive to the skin, prevent output from leaking into the space between the skin and the adhesive. This is in contrast to some non-switchable pressure sensitive adhesives, which typically require a significant amount of time, such as 10-60 minutes, to achieve strong adhesion. By applying pressure to the pressure sensitive adhesive it is possible for the adhesive to wet and flow faster into the skin surface, hereby obtaining a large contact area and hereby increasing the adhesive power. Some current adhesive systems for attachment of ostomy devices to the skin require a high or prolonged pressure from the user in order to sufficiently flow into and wet the surface of the substrate. By using a switchable adhesive with an initial high tack, neither a high pressure nor a long time is needed in order to ensure a good and enduring adhesion to the skin.

The quick and strong initial bonding of the switchable adhesive is thought to be at least partly the result of the ability of the switchable adhesive to quickly wet the skin, meaning that it will quickly flow into both the macro- and micro-structures of the skin and thereby establish a large contact surface between the adhesive and the skin. This flowing of the adhesive into the skin is a common phenomenon for pressure sensitive adhesives, but the speed at which it happens, and hence how quickly a strong adhesive bond is formed, varies widely for different compositions.

Typically, ostomy users will want to be able to move around shortly after having applied the ostomy device to the skin. Such movements can increase the risk of leakage if the adhesive has not yet achieved strong adhesion to the skin. With a switchable adhesive composition the risk of such leakage soon after application is reduced because of the rapidly forming strong adhesive bond.

In addition to the early formation of a strong adhesive bond, the switchable adhesive also makes it possible to maintain a very strong adhesive bond during the entire period of use of the ostomy device. This is because it is not necessary to be able to remove the adhesive in the pre-switched state. Therefore, the adhesion to the skin in the pre-switched state can remain very high right up until the switch causes the adhesion to drop significantly, thus allowing easy and pain-less removal of the device.

In embodiments, the first switchable adhesive composition is in contact with the backing layer. The first switchable adhesive composition may be disposed on the backing layer or coated on the backing layer. By being in contact with the backing layer, at least part of the switchable adhesive composition is close to the distal non-skin-facing surface of the adhesive wafer. This will make it easier to effect the switch of the switchable adhesive composition, for instance by applying light to the switchable adhesive composition through the backing layer.

In embodiments, the release liner is in contact with both the first switchable adhesive composition and the second absorbent adhesive composition. The release liner covers the surface of the adhesive that is to be attached to the skin of the user. As such, the surface of the adhesive that is in contact with the release liner is also the surface that will be in contact with the skin of the user during use. By having both the first switchable adhesive composition and the second absorbent adhesive composition form part of the adhesive surface that comes into contact with the user's skin, it is ensured that both adhesives can exert their respective effects directly on the skin. In other words, both adhesive compositions will be in contact with the user's skin during use. The switchable adhesive composition can cause the rapid, strong, and enduring adhesion to the skin and the absorbent adhesive composition can ensure that moisture is effectively removed from the surface of the skin.

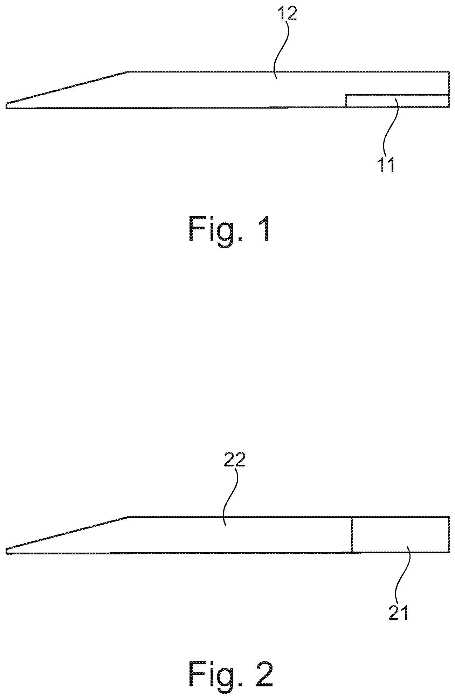

In figure 1 the second absorbent adhesive composition is located between the first switchable adhesive composition and the release liner. The absorbent adhesive composition can cover a part of the surface of the switchable adhesive composition on the proximal skin-facing side of the adhesive wafer. The switchable adhesive composition can cover the entire distal non-skin-facing surface of the absorbent adhesive composition. By such an arrangement, the absorbent adhesive will come into contact with the skin of the user and can thereby easily absorb moisture from the skin surface. None of the switchable adhesive will be covered on the distal non-skin-facing surface by the absorbent adhesive, thus making it easier to effect the switch by, for instance, shining light on the switchable adhesive without having to have the light pass through the absorbent adhesive.

In embodiments, the adhesive wafer has a central part adjacent to the hole for accommodating the stoma and a peripheral part adjacent to an edge of the adhesive wafer away from the hole. The second absorbent adhesive composition may be located in the central part of the adhesive wafer. The central part of the wafer is the part that is closer to the through-going hole in the wafer than it is to the peripheral edge of the wafer. Typically, this will represent a ring-shaped area of the adhesive wafer surrounding the hole. The central part will be the part of the wafer that is closest to the stoma during use of the ostomy device. The peripheral part is the remainder of the adhesive wafer outside the central part, i.e., the part that is closer to the peripheral edge than to the hole. Typically, the peripheral part will also be a ring-shaped area of the adhesive wafer. The absorbent adhesive may be in the entire central part of the wafer or only in part of the central part. The absorbent adhesive may extend also to the peripheral part of the adhesive wafer. By being in the central part of the adhesive wafer, the absorbent adhesive will be located close to the stoma and will thereby be close to the sensitive skin surrounding the stoma. This will allow the absorbent adhesive to absorb moisture form the sensitive skin around the stoma. Also, an absorbent adhesive may be able to swell during use as a consequence of the absorption of moisture and may thus be able to increase in volume and provide a mechanical sealing around the stoma.

In embodiments, the second absorbent adhesive composition is located only in the central part of the adhesive wafer. The absorbent adhesive composition may be located as a ring-shaped element in the central part of the adhesive wafer, thus surrounding the stoma during use.

In embodiments, the first switchable adhesive composition is located at least in the peripheral part of the adhesive wafer. The switchable adhesive composition may be in the entirety of the peripheral part or only in part of the peripheral part of the wafer. The switchable adhesive composition may extend into the central part of the wafer.

In embodiments, the releaser liner is in contact with the first switchable adhesive composition in the peripheral part of the adhesive wafer. In this manner, the switchable adhesive will be in contact with the skin at the peripheral part of the adhesive wafer during use. By having the switchable adhesive composition in the peripheral part in contact with the skin during use, a strong adhesive bond is established around the periphery of the adhesive wafer, thus making it less likely that the adhesive wafer will start to peel off due to contact with the surroundings, such as the user's clothes.

In embodiments, the releaser liner is in contact with the second absorbent adhesive composition in the central part of the adhesive wafer. In this manner, the absorbent adhesive will be in contact with the skin surrounding the stoma during use. This will allow the absorbent adhesive to absorb moisture directly from the sensitive skin surrounding the stoma, thereby preventing damage to the skin, such as maceration.

In embodiments, the backing layer is suitably elastic, i.e. it has a low modulus, enabling the adhesive construction to conform to the skin movement and provide comfort when using it. The backing layer may have a structured surface to improve the adhesion between the adhesive and the backing layer. The backing layer may be a non-woven or a non-woven-film laminate. The backing layer may be a polymer film. The backing layer may comprise polyurethane. The thickness of the backing layer is dependent on the type of backing layer used. For polymer films, such as polyurethane films, the overall thickness may be between 10 to 100 micrometers, such as between 10 to 50 micrometers, such as about 30 micrometers.

The release liner may be of any material known to be useful as a release liner for medical devices. For instance, the release liner may be in the form of a polymer film, foil, or paper, having release properties that enable the adhesive to be released easily from the liner. Such properties may be inherent in the material or the layer may be siliconized, coated with a low surface tension coating, or subjected to other appropriate surface modifications. Release liners are in general made on a mechanically stiff backing such as paper, polyethylene, polypropylene, or polyethylene terephthalate. This stiffness will support the adhesive wafer when applying the collecting device.

In embodiments, the second absorbent adhesive composition is in the form of a ring-shaped adhesive element located around the hole in the adhesive wafer and in contact with the release liner. Such a ring-shaped absorbent adhesive element could have a diameter of 30-70 mm, such as 40-70 mm, such as 50-70 mm, such as 60-70 mm. The ring-shaped adhesive element could for instance have a diameter of 30 mm, 40 mm, 50 mm, 60 mm, or 70 mm. The ring-shaped element could have a width, i.e. the distance from the inner rim of the ring to the outer rim of the ring measured along the surface of the ring, of at least 10 mm, at least 20 mm, at least 30 mm, at least 40 mm, at least 50 mm, 10-20 mm, 10-30 mm, 10-50 mm, 20-30 mm, 20-40 mm, 20-50 mm, 30-40 mm, 30-50 mm, or 40-50 mm. The width of the element can be constant over the entire element or it may vary.

In figure 1, the first switchable adhesive composition extends in the entire area of the adhesive wafer. In embodiments, the first switchable adhesive composition is in the form of a ring-shaped adhesive element located at the periphery of the adhesive wafer. Such a ring-shaped switchable adhesive element could have a diameter of 50-150 mm, such as 50-120 mm, such as 50-100 mm, such as 50-75 mm. The ring-shaped adhesive element could for instance have a diameter of 50 mm, 60 mm, 70 mm, 80 mm, 90 mm, 100 mm, 120 mm, or 150 mm. The ring-shaped element could have a width of at least 10 mm, at least 20 mm, at least 30 mm, at least 40 mm, at least 50 mm, at least 60 mm, at least 70 mm, at least 80 mm, at least 90 mm, at least 100 mm, 10-20 mm, 10-30 mm, 10-50 mm, 10-100 mm, 20-30 mm, 20-40 mm, 20-50 mm, 20-100 mm, 30-40 mm, 30-50 mm, 30-100 mm, 40-50 mm, 40-100 mm, or 50-100 mm. The width of the element can be constant over the entire element or it may vary.

An adhesive element could also have an only roughly ring-shaped, oval, or roughly oval form. In that case, the mentioned diameters would be the maximum distance from one point on the outer edge of the element to another point on the outer edge of the element.

In embodiments, the second absorbent adhesive composition has a uniform thickness, i.e. the distance from one outer surface of the adhesive to the other outer surface of the adhesive measured in a straight line perpendicular to the surface of the adhesive. In embodiments, the uniform thickness of the absorbent adhesive composition is at least 50 micrometers, such as at least 100 micrometers, such as at least 200 micrometers, such as at least 300 micrometers, such as at least 400 micrometers, such as at least 500 micrometers, such as at least 750 micrometers, such as at least 1,000 micrometers, such as at least 1,500 micrometers, such as at least 2,000 micrometers. The uniform thickness of the absorbent adhesive composition may be between 50 micrometers and 1,000 micrometers, such as 100-500 micrometers, such as 200-400 micrometers, such as 200-300 micrometers. The uniform thickness of the absorbent adhesive composition may be 50-250 micrometers, 100-250 micrometers, 250-500 micrometers, 250-750 micrometers, 500-750 micrometers, 500-1,000 micrometers, 500-1,500 micrometers, 500-2,000 micrometers, 1,000-1,500 micrometers, 1,000-1,500 micrometers, 1,000-2,000 micrometers, or 1,500-2,000 micrometers.

In embodiments, the second absorbent adhesive composition has a varied thickness. In embodiments, the second absorbent adhesive composition has maximum thickness of at least 50 micrometers, such as at least 100 micrometers, such as at least 200 micrometers, such as at least 300 micrometers, such as at least 400 micrometers, such as at least 500 micrometers, such as at least 750 micrometers, such as at least 1,000 micrometers, such as at least 1,500 micrometers, such as at least 2,000 micrometers. The maximum thickness of the absorbent adhesive composition may be between 50 micrometers and 1,000 micrometers, such as 100-500 micrometers, such as 200-400 micrometers, such as 200-300 micrometers. The maximum thickness of the absorbent adhesive composition may be 50-250 micrometers, 100-250 micrometers, 250-500 micrometers, 250-750 micrometers, 500-750 micrometers, 500-1,000 micrometers, 500-1,500 micrometers, 500-2,000 micrometers, 1,000-1,500 micrometers, 1,000-1,500 micrometers, 1,000-2,000 micrometers, or 1,500-2,000 micrometers.

In embodiments, the first switchable adhesive composition has a uniform thickness. In embodiments, the uniform thickness of the absorbent adhesive composition is at least 10 micrometers, such as at least 25 micrometers, such as at least 50 micrometers, such as at least 100 micrometers, such as at least 200 micrometers, such as at least 300 micrometers, such as at least 400 micrometers, such as at least 500 micrometers, such as at least 750 micrometers, such as at least 1,000 micrometers. The uniform thickness of the absorbent adhesive composition may be between 10 micrometers and 1,000 micrometers, such as 25-500 micrometers, such as 50-500 micrometers, such as 100-500 micrometers, such as 200-400 micrometers, such as 200-300 micrometers. The uniform thickness of the absorbent adhesive composition may be 10-50 micrometers, 10-100 micrometers, 25-50 micrometers, 25-100 micrometers, 50-100 micrometers, 50-250 micrometers, 100-250 micrometers, 250-500 micrometers, 250-750 micrometers, 500-750 micrometers, 500-1,000 micrometers.

In embodiments, the first switchable adhesive composition has a varied thickness. In embodiments, the maximum thickness of the absorbent adhesive composition is at least 10 micrometers, such as at least 25 micrometers, such as at least 50 micrometers, such as at least 100 micrometers, such as at least 200 micrometers, such as at least 300 micrometers, such as at least 400 micrometers, such as at least 500 micrometers, such as at least 750 micrometers, such as at least 1,000 micrometers. The maximum thickness of the absorbent adhesive composition may be between 10 micrometers and 1,000 micrometers, such as 25-500 micrometers, such as 50-500 micrometers, such as 100-500 micrometers, such as 200-400 micrometers, such as 200-300 micrometers. The maximum thickness of the absorbent adhesive composition may be 10-50 micrometers, 10-100 micrometers, 25-50 micrometers, 25-100 micrometers, 50-100 micrometers, 50-250 micrometers, 100-250 micrometers, 250-500 micrometers, 250-750 micrometers, 500-750 micrometers, 500-1,000 micrometers. In embodiments, the first switchable adhesive composition is thicker in the peripheral part of the adhesive wafer than in the central part of the adhesive wafer. In embodiments, a thickness of the first switchable adhesive composition in the peripheral part of the adhesive wafer is at least 120%, such as at least 150%, such as at least 200%, such as at least 250%, such as at least 500% of a thickness of the first switchable adhesive composition in the central part of the adhesive wafer.

In figure 1, the switchable adhesive composition is disposed on the backing layer and covers the entire backing layer. The absorbent adhesive composition is in the form of a ring-shaped adhesive element in the center of the adhesive wafer around the hole and on the skin-facing surface of the switchable adhesive composition. In this manner, the switchable adhesive composition will be in contact with the release liner in the periphery of the wafer and the absorbent adhesive composition will be in contact with the release liner in the center of the wafer. Both adhesives will therefore be in contact with the skin of the user during use.

In embodiments, the first switchable adhesive composition comprises curable molecules selected from the group consisting of acrylic acid esters or methacrylic acid esters of alcohols, glycols, pentaerythritol, trimethylpropane, glycerol, aliphatic epoxides, aromatic epoxides including bisphenol A epoxides, aliphatic urethanes, silicones, polyesters and polyethers.

In embodiments, the first switchable adhesive composition comprises a polymer selected from the group consisting of polyacrylates, polyurethanes, and polysilicones.

In embodiments, the first switchable adhesive composition comprises a photoinitiator. A photoinitiator makes it possible to switch the adhesive composition by activating the photoinitiator with light. Different photoinitiators have different absorption spectra and will need to be activated by light in different wavelengths. In embodiments, the first switchable adhesive composition comprises a photoinitiator reactive to visible light. This will make it possible to cause the switch of the adhesive by applying regular visible light. This is a safe and convenient method of switch, especially if the switch is to be effected by the user of the ostomy device.

In some embodiments, the light comprises visible light and/or ultraviolet (UV) light. Visible light is defined as electromagnetic radiation with a wavelength in the range 400-700 nm. Ultraviolet light is defined as electromagnetic radiation with a wavelength in the range 10-400 nm. In embodiments, the photoinitiator will be reactive to ultraviolet light.

In embodiments, the first switchable adhesive composition comprises a photoinitiator selected from the group consisting of titanocene photoinitiators; dye/co-initiator systems including thionine/triethanolamine; dye/borate salt systems; dye/peroxide systems and 1,2-diketone/co-initiator systems, including camphor-quinone/tertiary amine.

In embodiments, the switchable adhesive composition may be absorbent as described herein for the absorbent adhesive composition.

In embodiments, the switchable adhesive composition is a switchable pressure sensitive adhesive (PSA) composition. The switchable PSA may comprise a mixture, in proportions by weight, of 2% to 80% of curable molecules that are curable by free radical polymerisation, 0.05% to 10% of photoinitiator and an internal cross-linker that is crosslinkable by mechanism other than free radical polymerisation for cross linking the adhesive, the balance being base adhesive polymer and incidental constituents and the weight proportions being calculated on the basis of the dry weight of the base adhesive polymer. The PSA may have a cohesive strength of between 5 and 100 N/12.7×12.7 mm measured according to FINAT test method No. 18 The cohesive strength may be significantly higher than 30N/12.7×12.7 mm depending on the application for which the switchable PSA is intended. Preferably, the base adhesive polymer and curable molecules are mutually soluble when dry, although good results are still obtained when the curable molecules are uniformly dispersed in the adhesive even when the adhesive and curable molecules are mutually insoluble or only partly mutually soluble when dry.

The cohesive strength of the composition is determined by controlling the cohesive strength of the adhesive polymer backbone, and this is done by partially cross-linking it.

Cross-linking can be achieved by incorporating monomers of e.g. N-methylol acrylamide, N-(iso-butoxymethylene)acrylamide, methyl acrylamidoglycolate methyl ether (all 0.5-5%) or metal chelates, e.g., acetylacetonates of Zr, Al, or Fe (up to 2% of polymer weight) into the polymer backbone which then cross-links during drying after spreading on a substrate.

Al and Ti acetylacetonates and similar compounds can also be added after the polymerization step in concentrations between 0.1 and 2% of the polymer weight and used as an internal cross-linker through utilizing carboxylic groups in the polymer backbone during the drying step.

Multi functional isocyanates like toluene diisocyanate (TDI), trimethyl hexamethylene diisocyanate (TMDI), hexamethylene diisocyanate (HDI), or isophorane diisocyanate (IPDI), can be used to chemically inter link hydroxylic or carboxylic functions of different polymer chains, added in concentrations up to about 1% of the polymer weight.

Internal cross-linking can also be achieved between the carboxylic groups in the polymer backbone and added amino resins such as melamine, benzoguanamine, glycoluril, urea derivatives e.g. hexamethoxymethyl melamine, methoxymethyl methylol melamine, methoxymethyl ethoxymethyl benzoguanamine, tetrabutoxymethyl glycoluril, butoxymethyl methylol urea (up to 6%).

The above mentioned cross-linking can also be achieved using polycarbodiimides or multifunctional propylene imines.

It is also possible to blend one or more polymers having high cohesive strength with one or more polymers having low cohesive strength in order to achieve the desired balance.

Cross-linking is also important for effective switching and it is therefore necessary to distinguish between the type of cross-linking that is undertaken for controlling the cohesive strength of the adhesive composition and the type of cross-linking that brings about switching. In the first case, cross-linking for controlling the cohesive strength of the adhesive is effected using an internal cross-linker, i.e., a cross-linker supplied with or forming part of the adhesive polymer backbone material. In the second case, cross-linking for switching is effected by visible light or UV-induced curing of the curable molecules to form a three-dimensional polymeric network entangling the chains of the base adhesive polymer backbone, thereby reducing their mobility and free volume. Preferably the amount of base adhesive polymer present in the mixture is in the range 20% to 98% by weight, more preferably 40% to 90% by weight, and most preferably 50% to 70% by weight. Preferably the proportion of curable molecules in the mixture ranges from 2% to 80% by weight, more preferably 10% to 60% by weight, and most preferably 30% to 50% by weight. Preferably, the photoinitiator is present in the mixture in the proportions 0.1% to 5% by weight, more preferably 0.5% to 2% by weight. Preferably, the photoinitiator is also soluble in the dry mixture of adhesive and curable molecules, although it will be capable of exerting its curing initiating effect upon exposure to an activating light source if finely dispersed through the dry mixture but not dissolved in it.

The weight proportion for the base adhesive polymer is given here in terms of its dry weight and excludes any solvent which might normally be present in a commercially available bulk adhesive.

In certain embodiments, the weight proportion of base adhesive polymer is from one of the following lower endpoints (inclusive), or from one of the following upper endpoints (inclusive). The lower endpoints are 20%, 30%, 40%, 50%, 60% and 70%; the upper endpoints are 98%, 95%, 90% and 85%. In certain embodiments, the weight proportion of curable molecules is from one of the following lower endpoints (inclusive), or from one of the following upper endpoints (inclusive). The lower endpoints are 2%, 5%, 10% and 15%; the upper endpoints are 80%, 70%, 60%, 50%, 40% and 30%. In certain embodiments, the weight proportion of photoinitiator is from one of the following lower endpoints (inclusive), or from one of the following upper endpoints (inclusive). The lower endpoints are 0.05%, 0.1%, 0.2%, 0.5% and 1.0%; the upper endpoints are 10%, 5%, 4% and 3%.

The incidental constituents may be one or more of stabilizers, tackifiers, light scattering particles, fungicides, colorants, humectants, etc.

The adhesive component may be a hydrocolloid having polymeric chains extending from a core or nucleus, and the reference to the adhesive and the curable molecules being mutually soluble in each other when dry is to be understood as meaning that the curable molecules and the polymeric chains are mutually soluble in each other. Hydrocolloid-based medical dressings may be used for skin and wound treatment. When first attached to the skin, dry hydrocolloids are only slightly adherent to the skin, but quickly absorb moisture from the skin and become more tacky.

The preparation method for the switchable adhesive compositions is very simple. The adhesive component, the curable molecules (monomers and/or oligomers) and the photoinitiator are mixed, preferably stirred, together in darkness or under red light conditions for about 30 to 60 minutes, most conveniently at room temperature. The mixture also includes the internal cross-linker. The internal cross-linker may be included as part of the base adhesive, for example obtained from a commercial supplier who supplies as a stock item base adhesive with internal cross-linkers. Alternatively, the internal cross-linker may be supplied as a separate component from the base adhesive. The internal cross-linker may be added to the mixture as a solution. The adhesive component is usually supplied in solution (typically, 40% to 60% solids by weight); the solvent for the adhesive may be a suitable vehicle for dissolving the internal cross-linker. The curable molecules are usually solvent free, although some curable molecules of high viscosity may be carried in a solvent which also could act to stabilize the internal cross-linker; the photoinitiator is usually solid and the most difficult component of the system to dissolve and/or disperse.

Following completion of the mixing together, the resulting composition is spread onto, e.g., a release liner at a certain thickness-typically about 60 $\mu$m when wet-and then left to dry at room temperature for about 10 minutes. The release liner may be a polyethylene coated paper with a silicone compound chemically bound to the surface. The spread adhesive is then further dried at 80-150° C. for 3 to 10 minutes. A slightly higher temperature and a longer drying time can be used if necessary. After drying, the thickness of the spread adhesive will typically be about 30 $\mu$m.

The dried adhesive is then transferred onto a carrier film, for example, for peel strength and switching evaluation.

Alternatively, the dried adhesive may be transferred to a material for a wound dressing, for example a web of polyethylene or polyurethane film which may optionally be perforated, or a woven or non-woven fabric.

For a medical dressing or similar application, the adhesive component may be selected from polymers capable of forming shaped bodies, thin walls or coatings. Suitable polymers are biologically and pharmaceutically compatible, hypoallergenic and insoluble in and compatible with body fluids or tissues with which the dressing is contacted.

Exemplary light transmitting materials for carrying the adhesive polymer layer include polyethylene, polypropylene, polyurethane, ethylene/propylene copolymers, ethylene/ethylacrylate copolymers, ethylene/vinyl acetate copolymers, silicone elastomers, especially the medical-grade polydimethylsiloxanes, neoprene rubber, polyisobutylene, polyacrylates, chlorinated poly-ethylene, polyvinyl chloride, vinyl chloride-vinyl acetate copolymer, cross-linked polymethacrylate polymers (hydrogel), polyvinylidene chloride, poly(ethylene terephthalate), butyl rubber, epichlorohydrin rubbers, ethylenevinyl alcohol copolymers, ethylene-vinyloxyethanol copolymers; silicone copolymers, for example, polysiloxane-polycarbonate copolymers, polysiloxanepolyethylene oxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxane-ethylenesilane copolymers), and the like; cellulose polymers, for example methyl or ethyl cellulose, hydroxy propyl methyl cellulose, and cellulose esters; polycarbonates; polytetrafluoro-ethylene; and the like.

The adhesives may be water-soluble, but will most often be soluble in, and hence commercially supplied as solutions in, organic solvents such as ethyl acetate, hexane, toluene, acetone etc. Preferred adhesives are polyacrylates, poly-urethanes and polysilicones. Especially preferred are polyacrylates. By the term polyacrylates is meant acrylate, methacrylate and acrylate copolymer adhesives. Indeed acrylate copolymer adhesives are most preferred, e.g. alkyl acrylate copolymers. The most commonly used monomers in polyacrylates are butyl acrylate, ethylhexyl acrylate, hydroxyethyl acrylate and acrylic acid. They may be used singly or in a mixture, their relative proportions in the mixture being selected depending on the water penetration rate, viscoelastic properties, Tg, etc., that it is desired to achieve.

Cross-linking can be achieved by incorporating monomers of e.g. N-methylol acrylamide, N-(iso-butoxymethylene)acrylamide, methyl acrylamidoglycolate methyl ether (all 0.5-5%) or metal chelates, e.g., acetylacetonates of Zr, Al, or Fe (up to 2% of polymer weight) into the polymer backbone which then cross-links during drying after spreading on a substrate.

Al and Ti acetylacetonates and similar compounds can also be added after the polymerization step in concentrations between 0.1 and 3% of the polymer weight and used as an internal cross-linker through utilizing carboxylic groups in the polymer backbone during the drying step.

Multi functional isocyanates like TMDI, hexamethylene diisocyante, can be used to chemically inter link hydroxylic or carboxylic functions of different polymer chains, added in concentrations up to 5%, for example 1%, of the polymer weight.

Internal cross-linking can also be achieved between the carboxylic groups in the polymer backbone and added amino resins such as melamine, benzoguanamine, glycoluril, urea derivatives e.g. hexamethoxymethyl melamine, methoxymethyl methylol melamine, methoxymethyl ethoxymethyl benzoguanamine, tetrabutoxymethyl glycoluril, butoxymethyl methylol urea (up to 6%).

The above mentioned cross-linking can also be achieved using polycarbodiimides or multifunctional propylene imines.

The backbone adhesive polymer used as the adhesive component of the composition must include a functional group that is able to react chemically or physico-chemically with the internal cross-linker. It is also possible to use, as the starting or base adhesive, one which is manufactured with bound-in curable molecules; this is mixed with further curable molecules (not bound-in). The mechanism of internal cross-linking must not be a free radical mechanism because that is the mechanism used for effecting cross-linking for the switching.

Preferably, the curable molecules and the adhesive are soluble in each other when in the dry state, i.e., in the absence of a solvent. Alternatively, in the case that the adhesive and the curable molecules are not mutually soluble in each other when dry, or are only partly mutually soluble, they are uniformly dispersed in the composition. Typically, the adhesive (or the base adhesive if a mixture of adhesives is used) will be selected from polyacrylates, polyurethanes and silicone adhesives.

In the broadest sense, any conventional known unsaturated compounds could be used as the curable molecules, but preferred examples, used alone or in mixtures, are curable molecules such as acrylic acid esters or methacrylic acid esters of alcohols, glycols, pentaerythritol, trimethylpropane, glycerol, aliphatic epoxides, aromatic epoxides including bisphenol A epoxides, aliphatic urethanes, silicones, polyesters and polyethers, as well as ethoxylated or propoxylated species thereof.

The curable molecules have more than one unsaturated site, i.e., greater than single functionality. Multiple functionalities of 3 or greater, or more preferably 4 or greater are especially effective because curable molecules of this type are able to form highly cross-linked three-dimensional polymeric networks which are an important feature of switching, as will be explained below. Also, many curable molecules having multiple functionalities are commonly available at reasonable cost.

The radical initiator may be any species which is capable of producing radical species under the desired conditions but preferred examples are photoinitiators able to start the radical reaction under mild conditions, e.g. visible light, in order to promote radical polymerization reactions in the curable molecules. As a consequence, when the photoinitiator becomes activated by exposure to visible light, the curable molecules form chemical bonds with other curable molecules and hence create polymeric cross-linking. The effect of such cross-linking is to build a three-dimensional polymeric network entangling the adhesive polymer chains, thereby reducing their mobility and free volume. The photoinitiator may alternatively produce radical species under the mild conditions of long wave UV.

Curable molecules having multiple functionality are able to form highly cross-linked three-dimensional polymeric networks easily and hence exhibit good switching properties. The adhesive strength of the adhesive becomes reduced and it becomes less tacky so that it may be peeled more easily from the surface to which it is attached.

The adhesive mixture preferably also contains stabilizers which are added in order to prevent spontaneous cross-linking of the curable molecules during storage. Examples of such stabilizers are hydroquinones such as 4-methoxy phenol (sometimes referred to as hydroquinone monomethyl ether) and 2,4-ditert-butyl-metoxyphenol, or 1-piperidinyloxy-4,4'-[1,10-dioxo-1,10-decanediyl)bis(oxy)]bis[2,2,6,6-tetra methyl] and pentaerythritol tetrakis(3-(3,5-di-tert-butyl-4-hydroxyphenyl)propionate).

The adhesive mixture may also include photo-sensitisers. Since a sensitising species often absorbs energy in a different part of the spectrum from the initiator, more effective use of the light source may be achievable through the incorporation of sensitisers into the mixture. Many photo-sensitisers are complex organic molecules, absorbing in the visible portion of the spectrum.

The adhesive mixture may also incorporate light scattering particles to increase the effect of irradiation of the adhesive mixture. Preferably, the light scattering particles are an inorganic compound such as silica powder, alumina powder, silica-alumina powder or mica powder with particle sizes of the order of 10 nm or greater, typically up to 1 $\mu$m.

Any conventionally known free radical initiators may be used. Particularly preferred are those initiators which react to visible light radiation, although initiators which react under shorter wavelength light may be used in the compositions, depending on the application. Thus, free radical initiators which may be mentioned include titanocene photoinitiators; dye/co-initiator systems, e.g., thionine/triethanol-amine; dye/borate salt systems; dye/peroxide systems and 1,2-diketone/co-initiator systems, e.g., camphorquinone/tertiary amine.

Examples of visible light photoinitiators (which include Irgacure 784 because it absorbs light both in the UV and visible spectrum) are: Benzildimethyl ketal; Phenanthrenequinone; Titanocenes (of which Irgacure 784 is one example); Bis(2,4,6-trimethyl-benzoyl)-phenylphosphineoxide.

Examples of UV photoinitiators are: Benzoin and ethyl, isopropyl or isobutyl ethers of Benzoin; Benzophenone and hydroxy or methyl benzophenones; 2-Methyl-1[4-(methylthio)phenyl]-2-morpholinopropan-1-one; Acetophenone and 4'-Phenoxyacetophenone; Benzoyl-biphenyl; Benzil; Anisoin, as well as the Irgacures such as Irgacure 651 (benzyl dimethyl ketal) or Irgacure 907 (2-methyl-1-[4-(methylthio)phenyl]-2-morpholino-propan-1-one); or the Uvatones, such as Uvatone 8302 (2,2-diethoxy-1,2-diphenyl ethanone).

Preferred free radical photoinitiators for medical applications are the titanocene initiators such as bis.(.eta.5-cyclopentadienyl)-bis(2,6-difluoro-3-[pyrrol-1-yl]-phenyl) titanium, sold in the UK by Ciba Geigy as Irgacure 784 (Trade Mark).

In embodiments, the second absorbent adhesive composition comprises a polymer comprising monomer units selected from the group consisting of styrene, isoprene, butadiene, ethylene, and butylene.

In embodiments, the second absorbent adhesive composition comprises a styrene block co-polymer.

In embodiments, the second absorbent adhesive composition comprises a styrene block co-polymer selected from the group consisting of styrene-isoprene-styrene (SIS), styrenebutadiene-styrene (SBS), styrene-isobutylene-styrene (SIBS), and styrene-ethylene/butylene-styrene (SEBS).

In embodiments, the second absorbent adhesive composition comprises a polyethylene copolymer.

In embodiments, the second absorbent adhesive composition comprises a polyethylene copolymer selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide, and combinations thereof.

In embodiments, the second absorbent adhesive composition comprises polyisobutylene (PIB).

In embodiments, the absorbent adhesive composition may be switchable as described herein for the switchable adhesive composition.

In embodiments, the second absorbent adhesive composition comprises absorbent material. In embodiments, the second absorbent adhesive composition comprises water absorbent material.

In embodiments, the second absorbent adhesive composition comprises absorbent material selected from the group consisting of hydrocolloids, microcolloids, salt, and super absorbent particles.

In embodiments, the absorbent adhesive composition comprises an absorbent material in an amount of 1-60% (w/w) of the composition.

For instance, the absorbent adhesive composition comprises an absorbent material in an amount of 1-40% (w/w) or 1-20% (w/w) or 20-40% (w/w) or 20-60% (w/w) or 40-60% (w/w) or 25-50% (w/w) of the composition.

In embodiments, the absorbent material is selected from hydrocolloid, water soluble salt, mono, di- and oligosaccharides, sugar alcohols, polypeptides, organic acids, inorganic acids, amino acids, amines, urea, super absorbent particles such as polyacrylic acid, glycols such as polyethylene glycol, fumed silica, bentone, bentonite, and mixtures thereof.

In embodiments, the hydrocolloid is selected from guar gum, locust bean gum, pectin, potato starch, alginates, gelatine, xantan or gum karaya, cellulose derivatives, salts of carboxymethyl cellulose, sodium carboxymethyl cellulose, methyl cellulose, hydroxypropyl cellulose, hydroxyethyl cellulose, sodium starch glycolate, polyvinylalcohol, and mixtures thereof.

In embodiments, the water soluble salt is selected from $NaCl$, $CaCl_2$, $K_2SO_4$, $NaHCO_3$, $Na_2CO_3$, $KCl$, $NaBr$, $NaI$, $KI$, $NH_4Cl$, $AlCl_3$, $CH_3COONa$, $CH_3COOK$, $HCOONa$, $HCOOK$, and mixtures thereof.

In embodiments, the switchable and/or the absorbent adhesive composition may comprise ingredients such as tackifiers, extenders, non-reactive polymers, oils (e.g. polypropyleneoxide, ethyleneoxide-propyleneoxide copolymers, mineral oil), plasticizers, fillers, and surfactants.

In embodiments, the absorbent adhesive composition has an absorption of at least 0.05 $g/cm^3/2h$, measured as described herein, such as an absorption of at least 0.06, 0.07, 0.08, 0.09, 0.1, 0.2, 0.3, 0.4, 0.5, 0.6, 0.7, 0.8, 0.9, or 1 $g/cm^3/2h$ measured as described herein.

In embodiments, the first switchable adhesive composition and/or the second absorbent adhesive composition has a moisture vapor transmission rate (MVTR) above 250 $g/m^2/24h$ measured as described herein, such as above 500, 750, 1000, 1250, 1500, 2000, 2500, or 3000 $g/m^2/24h$ measured as described herein.

One element in forming the adhesive bond is the flow of the adhesive composition into the micro- and macro-structure of the substrate. The better the adhesive composition is able to flow into, i.e. wet, the substrate, the larger the adhesive contact area obtained. A large contact area between the adhesive and the substrate will lead to improved adhesion. Wetting of a substrate by an adhesive composition is dependent on the characteristics of the composition as well as upon, e.g., time, temperature, and pressure. In relation to wetting of a substrate, a central characteristic of an adhesive composition is the viscosity, measured herein as the complex viscosity $|\eta^*|$.

In embodiments, the switchable adhesive composition has a complex viscosity $|\eta^*|$ below 400,000 Pa s, 300,000 Pa s, 200,000 Pa s, below 150,000 Pa s, below 100,000 Pa s, below 75,000 Pa s, below 50,000 Pa s, below 25,000 Pa s, below 10,000 Pa s, below 5,000 Pa s, below 1,000 Pa s, below 500 Pa s, below 250 Pa s, below 100 Pa s, below 50 Pa s, or below 10 Pa s measured as described herein. In embodiments, the switchable adhesive composition has a complex viscosity $|\eta^*|$ of 10-50 Pa s, 50-100 Pa s, 100-250 Pa s, 250-500, Pa s, 500-1,000 Pa s, 1,000-5,000 Pa s, 5,000-10,000 Pa s, 10,000-25,000 Pa s, 25,000-50,000 Pa s, 50,000-75,000 Pa s, 75,000-100,000 Pa s, 100,000-150,000 Pa s, 150,000-200,000 Pa s, 200,000-300,000 Pa s, 300,000-400,000 Pa s, 400,000-500,000 Pa s, 10-100 Pa s, 100-1,000 Pa s, 1,000-10,000 Pa s, 10,000-100,000 Pa s, or 100,000-500,000 Pa s.

Complex viscosity is a measure of the resistance to gradual deformation of a given liquid state composition. Generally, the lower the viscosity, the more quickly the composition will be able to wet a rough surface by flowing into the small structures of the surface, such as the microstructure of skin.

In the present context, a relatively low complex viscosity is advantageous in that it will lead to the composition more easily and quickly flowing into the contour of the skin.

An advantage of this low viscosity is that the adhesive in the first state will be able to easily and quickly flow into, i.e. wet, the microstructure of the skin as well as larger irregularities, such as scar tissue and wrinkles. This means that a large contact surface between the adhesive and the skin is quickly established and that a good adhesive bond between the skin and the adhesive is quickly obtained.

In embodiments, the complex viscosity of the switchable adhesive composition after switch is at least 2 times, such as at least 5 times, such as at least 10 times, such as at least 20 times, such as at least 50 times, such as at least 100 times, such as at least 1,000 times, such as at least 10,000 times higher than the complex viscosity of the switchable adhesive composition before switch.

*Dynamic mechanical analysis (DMA) and determination of complex viscosity |η*|*

The parameter complex viscosity |η*| was measured as follows by a frequency sweep. The adhesives were pressed into a plate of 1 mm thickness. A round sample of 25 mm in diameter was cut out and placed in a Haake RheoStress 6000 rotational rheometer from Thermo Scientific. The geometry applied was parallel plates 25 mm and the shear stress was fixed at 5556 Pa and a gap size of 0.9-1.05 mm was applied to the sample in the beginning of the measurement to obtain a normal force of approximately 5 N. The measurements were carried out at 32°C. For the complex viscosity |η*| the value measured at a frequency of 0.01 Hz was used.

*Moisture vapour transmission rate*

Moisture vapour transmission rate (MVTR) is measured in grams per square meter ($g/m^2$) over a 24 hours period using an inverted cup method.

A container or cup that was water and water vapour impermeable having an opening of Ø35 mm was used. 20 mL saline water (0.9% NaCl in demineralised water) was placed in the container and the opening was sealed with the test adhesive mounted on a highly permeable polyurethane (PU) backing film (BL9601 foil from Intellicoat). The container was placed into an electrically heated humidity cabinet and the container or cup was placed upside down, such that the water was in contact with the adhesive. The cabinet was maintained at 32°C and 15% relative humidity (RH).

The weight loss of the container was followed as a function of time. The weight loss was due to water transmitted through the adhesive and/or film. This difference was used to calculate the MVTR of the test adhesive film. MVTR was calculated as the weight loss per time divided by the area of the opening in the cup ($g/m^2/24h$).

The MVTR of a material is a linear function of the thickness of the material. Thus, when reporting MVTR to characterize a material, it is important to inform the thickness of the material which MVTR was reported. We used 150 μm as a reference. If thinner or thicker samples were measured, the MVTR was reported as corresponding to a 150μm sample. Thus a 300 μm sample with a measured MVTR of 10 $g/m^2/24h$ was reported as having MVTR = 20 $g/m^2/24h$ for a 150 μm sample because of the linear connection between thickness of sample and MVTR of sample.

Finally, we noted that by using this method, we introduced an error by using a supporting PU film. Utilizing the fact that the adhesive/film laminate was a system of two resistances in series eliminated the error. When the film and the adhesive are homogeneous, the transmission rate may be expressed as:

$$1/P(measured) = 1/P(film) + 1/P(adhesive).$$

Hence, by knowing the film permeability and thickness of the adhesive, it was possible to calculate the true permeability of the adhesive, P(adhesive), using the following expression:

$$P(adhesive) = d(adhesive)/150\mu m * 1/(1/P(measured) - 1/P(film))$$

where d(adhesive) was the actual measured thickness of the adhesive and P(film) was the MVTR of the film without any adhesive on and P(measured) was the actual measured MVTR.

*Moisture absorption*

Samples were prepared by thermoforming to a 0.5 mm thick adhesive film between two release liners. With a punching tool, samples were punched out. Sample size was 25 x 25 mm. The release liners were removed. The samples were glued to an object glass and placed in a beaker with physiological salt water and placed in an incubator at 37 °C.

The sample was weighed at the outset (M(start)) and after 2 hours (M(2 hours). Before weighing, the object glass was dried off with a cloth. For a 25 x 25 mm sample the area was 6.25 $cm^2$ (the surface edges were left out of the area). The moisture absorption may be calculated as: Water absorption after 2 hours = (M(2 hours) - M(start))/6.25 $cm^2$. The result is in the unit $g/cm^2$ per 2 hours.

**Detailed Description of the Drawings**

Figure 1 is a schematic cross-section view of one half of the adhesives of an adhesive wafer not belonging to the invention. In this figure, a first adhesive 11 is placed in a recess in a second adhesive 12. The lower surface of the adhesive wafer is the surface that is in contact with the skin of the user during use. This surface may be covered by a release liner (not illustrated), which is removed prior to adhering the wafer to the skin. The upper surface is the surface facing away from the skin during use. This surface can be covered by a backing layer (not illustrated) to which the collecting bag is or can be attached. The outer edge of the adhesive wafer, which faces away from the stoma during use, is bevelled. In Fig. 1, the first adhesive 11 is placed in a recess on the skin-facing surface of the second adhesive 12. The first adhesive 11 is placed in the central part of the adhesive wafer, bordering the stoma during use. The first adhesive 11 can be a "first switchable adhesive composition" as described herein or a "second absorbent adhesive composition" as described herein. The second adhesive 12 can be a "first switchable adhesive composition" as described herein or a "second absorbent adhesive composition" as described herein.

Figure 2 is a schematic cross-section view of one half of the adhesives of an adhesive wafer. In this figure, the first adhesive 21 is placed adjacent to the second adhesive 22. The first adhesive 21 is placed centrally on the wafer, bordering the stoma during use. The second adhesive 22 is placed peripherally on the wafer. The outer edge of the adhesive wafer, which faces away from the stoma during use, is bevelled. The first adhesive 11 can be a "first switchable adhesive composition" as described herein or a "second absorbent adhesive composition" as described herein. The second adhesive 12 can be a "first switchable adhesive composition" as described herein or a "second absorbent adhesive composition" as described herein.

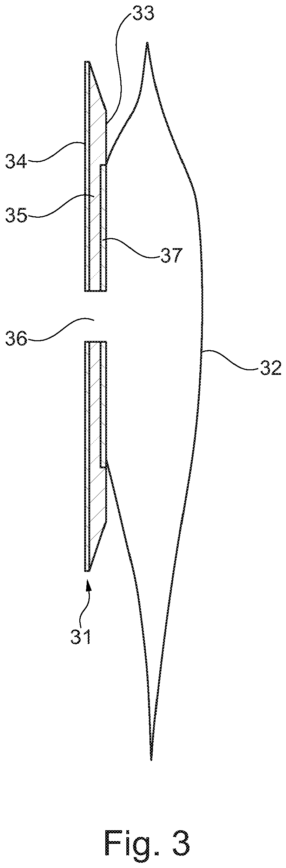

Figure 3 is a schematic cross-section view of an ostomy device, including an adhesive wafer 31 and a collecting bag 32. The adhesive wafer 31 has a centrally located through-going hole allowing output from the stoma (not illustrated) to pass into the collecting bag 32. The adhesive wafer 31 comprises a release liner 34, placed on the skin-facing side of the adhesive layer 35 and a backing layer 33 placed on the non-skin-facing surface of the adhesive layer 35. A further optional barrier layer 37 can be placed on top of the backing layer 33 in the area where the collecting bag 32 is attached to the adhesive wafer 31. In this figure, the adhesive layer 35 is illustrated as a single homogenous layer, but the adhesive layer 35 comprises a first switchable adhesive and a second absorbent adhesive as described herein. These adhesives may be arranged as illustrated in figures 1 and 2 or in any other manner.

Embodiments, and features of the various exemplary embodiments described in this application, may be combined with each other ("mixed and matched"), unless specifically noted otherwise.

## Examples

### *Preparation of adhesives*

### Absorbent adhesive

The absorbent adhesives composition is based on a commercial standard wear adhesive for ostomy care as provided, for instance, in the center adhesive of the SenSura® Mio ostomy device from Coloplast A/S.

The absorbent adhesive composition tested consists of Kraton 1161 (Kraton polymers), Oppanol B12 (BASF), pectin LM CG (CP Kelco), Akucell AF288 (Akzo Nobel), PB gelatin (PB Gelatins), and guar gum FG-20 (Hercules Corp). The components have been mixed in a z-blade Austin 300 g mixer, applied vacuum, and pressed into a 1 mm sheet and top film of linear low density polyethylene or similar placed on one side.

### Switchable adhesives

Four different switchable adhesives have been produced and tested. The adhesive recipes are provided below.

### *Composition 1: BASF acResin A 260 UV with 1% photoinitiator*

80 g BASF acResin A 260 UV was dissolved in 120 mL toluene at room temperature using a shaker with a speed of 30 rpm. 60 g of the resulting solution was mixed with 0.24 g Irgacure 784 photoinitiator using a spatula for 1 min.

### *Composition 2: BASF acResin A 260 UV with 1% photoinitiator and 25 % hydrocolloids*

For the compositions that were to contain 25% hydrocolloids, 2 g of hydrocolloid mixture (10% (w/w) pectin LM CG, CP Kelco, 20% (w/w) Akucell AF288, Akzo Nobel, 30% (w/w) PB gelatin, PB Gelatins, and 40 % (w/w) Guar gum FG-20, Hercules Corp.) was added to 15g of the mixture containing BASF acResin A 260 UV with 1% photoinitiator as described above.

*Composition 3: Aroset 1450 Z 40 based composition with 1% photoinitiator*

60 g of the Aroset 1450 Z 40 solution was mixed with 0.24 g Irgacure 784 photoinitiator using a spatula for 1 min.

*Composition 4: Aroset 1450 Z 40 based composition with 1% photoinitiator and 25% hydrocolloids*

2 g of hydrocolloid mixture (10 % (w/w) pectin LM CG,CP Kelco, 20% (w/w) Akucell AF288, Akzo Nobel ,30 % (w/w) PB gelatine, PB Gelatins and 40 % (w/w) Guar gum FG-20, Hercules Corp.) was added to 15 g of the mixture containing Aroset 1450 Z 40 compound, toluene and photoinitiator, and mixed for 1 min using a spatula.

### Example 1: Peel force before and after switch

### Preparation of samples

Following the quantities and steps of a recipe, the compounds are hand mixed in a dark glass recipient for 1-2 minutes. The mixtures are let to rest for 24h before use, the necessary time for the crosslinker to dissolve.
Afterwards the solution is coated with a dog-bone coater (f ex. using the 500 $\mu$m thickness), on a siliconized paper used as a release liner (RL). Before use, or before addition of top film (that can be polyurethane PU or polyethylene PE - both from the Mio top film product), the films are let for evaporation for a long enough time (48-72h). The final thickness of the samples varies between 120 and 170$\mu$m.
When HC were added the same coating procedure is applied, only that the thickness of the films will be different.

### Peel setup

To perform peel tests 90°, the samples with top film added (PU or PE) were cut in rectangular shapes (25x100mm) and a helping tape on top. For the uncured samples, an occlusive black film was added on top to protect them from curing; also fast and in-the-dark handling was performed.
A sample of 25x100 mm2 was cut from the adhesive and firmly pressed on to a thoroughly cleaned plate (HDPE or TEFLON). A 25x300 mm2 piece of auxiliary tape was then placed on the top of the adhesive and the whole sample pressure rolled to assure firm adhesion between the tape and the adhesive to be tested. After conditioning for 30 minutes at 23 plus or minus 3 degrees centigrade, the sample was mounted in a tensile testing machine and a 90 degrees peel test was carried out at a speed of 304 mm/min.

### Results

The results are given in the table below in the unit N/25 mm. The results clearly demonstrate that all four adhesive compositions exhibit high peel force before switch and lower peel force after switch. For all compositions, at least a 50% drop in peel force is observed when going from the non-switched to the switched composition.

Table 1: Results from 90 degree peel test

| Composition | Peel force, before switch (N/25mm) | Peel force, after switch (N/25mm) | Reduction in peel force |
|---|---|---|---|
| Composition 1 (BASF acResin with 1% PI) | 5.67 | 1.83 | 68% |
| Composition 2 (BASF acResin with 1% PI and 25% HC) | 7.89 | 3.76 | 52% |
| Composition 3 (Aroset 1450 with 1% PI) | 3.40 | 1.41 | 59% |
| Composition 4 (Aeroset with 1% PI and 25% HC) | 3.24 | 1.54 | 52% |

### Example 2: Fast-tack bending device test

Another way to measure leakage with time would be to measure a device leaking during simulated use containing simulated output.

**Product**

A product could be constructed of an absorbent hydrocolloid adhesive as described herein in the center (diameter 50 mm) and a switchable adhesive as described herein placed in the rim (diameter 100 mm). The resulting adhesive wafer could be attached to an ostomy bag. A center hole could be cut to diameter 30 mm.

A bending test construction is set up in such a way that a skin substrate is mounted on a soft silicone plate (20x20x2 cm, shore 2-15 A) with an acrylic glue. The soft silicone plate with the skin on, is mounted on two steel plates (10x20x0.5 mm) that can bend in such a way that the silicone plate will bend 40 degrees form horizontal in the middle of the plane. This bending test is carried out at a frequency of 30/min.

The ostomy product is placed on the top of the skin and 50 ml hand soap solution added to the bag. The test is done at ambient temperature, approximately 20°C for 10 min runtime. The dwell time of the ostomy product on the skin is varied in order to see the effect of leakage. Leakage is determined by visual inspection of the ostomy product.

**Expected results**

We would expect that, when testing right after application, the center absorbent hydrocolloid adhesive would not yet have bonded to the skin and the liquid will therefore be able to leak out at least to the edge of the absorbent adhesive and possibly somewhat into the peripheral switchable adhesive. This peripheral switchable adhesive will have bonded instantly and will therefore likely be able to withstand the liquid, i.e., not allow passage of liquid between the switchable adhesive and the skin. When waiting for the absorbent center adhesive to bond, e.g. after 10 min, leakage underneath the absorbent adhesive will likely not happen during the 10 min test period.

Table 2: Expected approximate leakage results for device

| Application time (min) | Dwell time before test (min) | Expected leakage in center (absorbent adhesive) | Expected leakage in periphery (switchable adhesive) |
|---|---|---|---|
| 5 | 0 | High | Low or none |
| 5 | 5 | Medium | Low or none |
| 5 | 10 | Low or none | None |

**Claims**

1. An ostomy device comprising an adhesive wafer (31) for attachment to a skin surface of a user, and a collecting bag (32) connected to the adhesive wafer (31); the adhesive wafer (31) having a through-going hole for accommodating the stoma of the user; and the adhesive wafer (31) comprising a backing layer (33), a first adhesive composition (21), a second absorbent adhesive composition (22), **characterised in that** the first adhesive composition (21) is a switchable adhesive composition and the ostomy device further comprises a release liner (34), wherein the adhesive wafer (31) has a central part adjacent to the hole for accommodating the stoma and a peripheral part adjacent to an edge of the adhesive wafer (31) away from the hole, and wherein the second absorbent adhesive composition (22) is located at least in the central part of the adhesive wafer (31), wherein the releaser liner (34) is in contact with the first switchable adhesive composition (11, 21) in the peripheral part of the adhesive wafer (31), and wherein the releaser liner (34) is in contact with the second absorbent adhesive composition (22) in the central part of the adhesive wafer (31).

2. The ostomy device according to claim 1, wherein the switchable adhesive composition (21) is switchable from a high-tack state with a first peel force to a lower-tack state with a second peel force.

3. The ostomy device according to claim 2, wherein the first peel force is higher than the second peel force.

4. The ostomy device according to claim 2 or 3, wherein the peel force is measured by the 90 degree peel test described herein.

5. The ostomy device according to any one of claims 2-4, wherein a reduction in peel force between the first peel force and the second peel force is at least 50%.

**6.** The ostomy device according to claim 5, wherein the reduction in peel force between the first peel force and the second peel force is 50-70%.

**7.** The ostomy device according to any one of the preceding claims, wherein the first switchable adhesive composition (21) is in contact with the backing layer (33).

**8.** The ostomy device according to any one of the preceding claims, wherein the second absorbent adhesive composition (22) is located between the first switchable adhesive composition (21) and the release liner (34).

**9.** The ostomy device according to any one of the preceding claims, wherein the first switchable adhesive composition (21) comprises a photoinitiator.

**10.** The ostomy device according to any one of the preceding claims, wherein the first switchable adhesive composition (21) comprises a photoinitiator reactive to visible light.

**11.** The ostomy device according to any one of the preceding claims, wherein the second absorbent adhesive composition (22) comprises absorbent material.

**12.** The ostomy device according to any one of the preceding claims, wherein the second absorbent adhesive composition (22) comprises absorbent material selected from the group consisting of hydrocolloids, microcolloids, salt, and super absorbent particles.

**13.** The ostomy device according to any one of the preceding claims, wherein the second absorbent adhesive composition (22) has an absorption of at least $0.05 \, g/cm^3/2h$, measured according to the moisture absorption test described herein.


**Patentansprüche**

**1.** Ostomie-Vorrichtung, umfassend ein Klebeplättchen (31) zur Befestigung an einer Hautoberfläche eines Benutzers, und einen Auffangbeutel (32), der mit dem Klebeplättchen (31) verbunden ist, wobei das Klebeplättchen (31) ein Durchgangsloch zur Aufnahme des Stomas des Benutzers aufweist; und wobei das Klebeplättchen (31) eine Trägerschicht (33), eine erste Klebstoffzusammensetzung (21), und eine zweite saugfähige Klebstoffzusammensetzung (22) umfasst, **dadurch gekennzeichnet, dass** die erste Klebstoffzusammensetzung (21) eine zustandsänderbare Klebstoffzusammensetzung ist und die Ostomie-Vorrichtung ferner eine Trennschicht (34) umfasst, wobei das Klebeplättchen (31) einen mittleren Teil neben dem Loch zur Aufnahme des Stomas und einen Umfangsteil neben einem Rand des Klebeplättchens (31) vom Loch entfernt aufweist, und wobei die zweite saugfähige Klebstoffzusammensetzung (22) mindestens im mittleren Teil des Klebeplättchens (31) angeordnet ist, wobei die Trennschicht (34) mit der ersten zustandsänderbaren Klebstoffzusammensetzung (11, 21) im Umfangsteil des Klebeplättchens (31) in Kontakt ist, und wobei die Trennschicht (34) mit der zweiten saugfähigen Klebstoffzusammensetzung (22) im mittleren Teil des Klebeplättchens (31) in Kontakt ist.

**2.** Ostomie-Vorrichtung nach Anspruch 1, wobei die zustandsänderbare Klebstoffzusammensetzung (21) von einem Zustand mit hoher Haftkraft mit einer ersten Abzugskraft in einen Zustand mit geringerer Haftkraft mit einer zweiten Abzugskraft zustandsänderbar ist.

**3.** Ostomie-Vorrichtung nach Anspruch 2, wobei die erste Abzugskraft höher als die zweite Abzugskraft ist.

**4.** Ostomie-Vorrichtung nach Anspruch 2 oder 3, wobei die Abzugskraft mit dem hierin beschriebenen 90-Grad-Abzugstest gemessen wird.

**5.** Ostomie-Vorrichtung nach einem der Ansprüche 2-4, wobei eine Verringerung der Abzugskraft zwischen der ersten Abzugskraft und der zweiten Abzugskraft mindestens 50% beträgt.

**6.** Ostomie-Vorrichtung nach Anspruch 5, wobei die Verringerung der Abzugskraft zwischen der ersten Abzugskraft und der zweiten Abzugskraft 50-70% beträgt.

**7.** Ostomie-Vorrichtung nach einem der vorhergehenden Ansprüche, wobei die erste zustandsänderbare Klebstoffzusammensetzung (21) mit der Trägerschicht (33) in Kontakt ist.

**8.** Ostomie-Vorrichtung nach einem der vorhergehenden Ansprüche, wobei die zweite saugfähige Klebstoffzusammensetzung (22) zwischen der ersten zustandsänderbaren Klebstoffzusammensetzung (21) und der Trennschicht (34) angeordnet ist.

**9.** Ostomie-Vorrichtung nach einem der vorhergehenden Ansprüche, wobei die erste zustandsänderbare Klebstoffzusammensetzung (21) einen Photoinitiator umfasst.

**10.** Ostomie-Vorrichtung nach einem der vorhergehenden Ansprüche, wobei die erste zustandsänderbare Klebstoffzusammensetzung (21) einen Photoinitiator umfasst, der auf sichtbares Licht reagiert.

**11.** Ostomie-Vorrichtung nach einem der vorhergehenden Ansprüche, wobei die zweite saugfähige Klebstoffzusammensetzung (22) ein saugfähiges Material umfasst.

**12.** Ostomie-Vorrichtung nach einem der vorhergehenden Ansprüche, wobei die zweite saugfähige Klebstoffzusammensetzung (22) ein saugfähiges Material umfasst, das aus der aus Hydrokolloiden, Mikrokolloiden und supersaugfähigen Teilchen bestehenden Gruppe ausgewählt ist.

**13.** Ostomie-Vorrichtung nach einem der vorhergehenden Ansprüche, wobei die zweite saugfähige Klebstoffzusammensetzung (22) eine Absorption von mindestens 0,05 g/cm$^3$/2h, gemessen nach dem hierin beschriebenen Feuchtigkeitsabsorptionstest, aufweist.


**Revendications**

**1.** Dispositif stomique comprenant une tranche adhésive (31), à fixer à la surface de peau d'un utilisateur, et un sac collecteur (32) raccordé à la tranche adhésive (31),
la tranche adhésive (31) comportant un trou traversant pour accueillir la stomie de l'utilisateur ; et
la tranche adhésive (31) comprenant une couche dorsale (33), une première composition adhésive (21) et une seconde composition adhésive absorbante (22), **caractérisé en ce que** la première composition adhésive (21) est une composition adhésive recollable et que le dispositif stomique comprend en outre un film de protection (34),
dans lequel la tranche adhésive (31) comporte une partie centrale adjacente au trou pour accueillir la stomie et une partie périphérique adjacente à un bord de la tranche adhésive (31) à l'écart du trou ; et
dans lequel la seconde composition adhésive absorbante (22) est située au moins dans la partie centrale de la tranche adhésive (31) ;
dans lequel le film de protection (34) est en contact avec la première composition adhésive (11, 21) recollable dans la partie périphérique de la tranche adhésive (31) ; et
dans lequel le film de protection (34) est en contact avec la seconde composition adhésive absorbante dans la partie centrale de la tranche adhésive (31).

**2.** Dispositif stomique selon la revendication 1, dans lequel la première composition adhésive (21) recollable peut être transférée d'un état à pégosité élevée ayant une première force de pelage à un état à pégosité inférieure ayant une seconde force de pelage.

**3.** Dispositif stomique selon la revendication 2, dans lequel la première force de pelage est plus élevée que la seconde force de pelage.

**4.** Dispositif stomique selon la revendication 2 ou 3, dans lequel la force de pelage se mesure par l'essai de pelage à 90 degrés décrit dans le présent document.

**5.** Dispositif stomique selon l'une quelconque des revendications 2 à 4, dans lequel la réduction de force de pelage entre la première force de pelage et la seconde force de pelage est d'au moins 50 %.

**6.** Dispositif stomique selon la revendication 5, dans lequel la réduction de force de pelage entre la première force de pelage et la seconde force de pelage est dans une plage 50-70 %.

**7.** Dispositif stomique selon l'une quelconque des revendications précédentes, dans lequel la première composition adhésive (21) recollable est en contact avec la couche dorsale (33) .

8.  Dispositif stomique selon l'une quelconque des revendications précédentes, dans lequel la seconde composition adhésive absorbante (22) est située entre la première composition adhésive (21) recollable et le film de protection (34).

9.  Dispositif stomique selon l'une quelconque des revendications précédentes, dans lequel la première composition adhésive (21) recollable comprend un photo-initiateur.

10. Dispositif stomique selon l'une quelconque des revendications précédentes, dans lequel la première composition adhésive (21) recollable comprend un photo-initiateur réagissant à la lumière visible.

11. Dispositif stomique selon l'une quelconque des revendications précédentes, dans lequel la seconde composition adhésive absorbante (22) comprend une matière absorbante.

12. Dispositif stomique selon l'une quelconque des revendications précédentes, dans lequel la seconde composition adhésive absorbante (22) comprend une matière absorbante choisie dans le groupe constitué par les hydrocolloïdes, les microcolloïdes, le sel et des particules super-absorbantes.

13. Dispositif stomique selon l'une quelconque des revendications précédentes, dans lequel la seconde composition adhésive absorbante (22) présente une absorption d'au moins 0,05 g/cm$^3$/2 h, mesurée selon l'essai d'absorption d'humidité décrit dans le présent document.

12

11

Fig. 1

22

21

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009006902 A1

- EP 2372920 A1